(19) Europäisches Patentamt
European Patent Office
Office européen des brevets

(11) **EP 3 064 268 B1**

(12) **FASCICULE DE BREVET EUROPEEN**

(45) Date de publication et mention
de la délivrance du brevet:
**25.10.2017  Bulletin 2017/43**

(51) Int Cl.:
***B01J 8/12*** (2006.01)

(21) Numéro de dépôt: **16305154.3**

(22) Date de dépôt: **11.02.2016**

(54) **ENSEMBLE DE COLLECTE D'UN FLUIDE GAZEUX POUR REACTEUR RADIAL**

SAMMELEINHEIT FÜR GASFÖRMIGES FLUID FÜR EINEN RADIALREAKTOR

ASSEMBLY FOR COLLECTING A GASEOUS FLUID FOR A RADIAL REACTOR

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.03.2015  FR 1551832**

(43) Date de publication de la demande:
**07.09.2016  Bulletin 2016/36**

(73) Titulaire: **IFP Énergies nouvelles
92852 Rueil-Malmaison Cedex (FR)**

(72) Inventeurs:
- **BAZER-BACHI, Frederic
  69540 IRIGNY (FR)**
- **PLAIS, Cecile
  69420 LES HAIES (FR)**
- **SANCHEZ, Eric
  69230 SAINT GENIS LAVAL (FR)**

(56) Documents cités:
**EP-A1- 0 483 975    FR-A1- 2 953 738
US-A- 3 909 208    US-A- 4 277 444**

## Description

La présente invention concerne un ensemble de collecte d'un fluide gazeux pour des unités en lit mobile présentant une circulation radiale de la charge qui implique un écoulement à travers un lit catalytique selon un ensemble de directions correspondant à des rayons orientés depuis la périphérie vers le centre, ou depuis le centre de l'enceinte vers la périphérie. La présente invention a également pour objet un réacteur à lit radial comprenant un ensemble de collecte d'effluent réactionnel gazeux. Enfin l'invention se rapporte à un procédé de conversion catalytique d'une charge d'hydrocarbures mettant en oeuvre un réacteur à lit radial.

## Etat de la technique

L'unité la plus représentative de ce type d'écoulement radial est le reformage régénératif des coupes hydrocarbures de type essences qu'on peut définir comme ayant un intervalle de distillation compris entre 80 et 250°C. Mais le domaine d'application de la présente invention est plus large et on peut citer en plus du reformage catalytique des essences, l'isomérisation squelettale de diverses coupes oléfiniques en C4, C5, ou encore le procédé de métathèse pour la production de propylène par exemple. Cette liste de procédé n'est pas exhaustive, et la présente invention peut s'appliquer à tout type de procédé catalytique à flux radial et charge gazeuse. Ainsi dans le cadre des nouvelles technologies de l'énergie, les procédés alcool vers alcène, par exemple, pourraient utiliser ce type de technologie.

Certaines de ces unités à lit radial, dont le reformage régénératif, font appel à un écoulement du catalyseur dit en lit mobile, c'est à dire un écoulement gravitaire lent des particules de catalyseur confinées (ou lit catalytique) dans un espace annulaire limité par la paroi du réacteur et une paroi intérieure perméable au gaz et imperméable aux grains de catalyseur qui correspond au conduit de collecte (ou collecteur central) qui récupère les effluents réactionnels.

Alternativement, le lit catalytique mobile peut être confiné dans un espace, généralement annulaire, formé entre une grille dite "extérieure" et le conduit de collecte qui sont agencés de préférence de manière concentrique. La grille dite "extérieure" peut être constituée par un assemblage d'éléments de grille en forme de coquilles (scallops selon la terminologie anglo-saxonne). La charge gazeuse est généralement introduite par la périphérie extérieure du lit annulaire et traverse le lit catalytique de manière sensiblement perpendiculaire à la direction verticale d'écoulement de ce dernier. Les effluents réactionnels sont ensuite récupérés dans le conduit de collecte (ou collecteur).

La mise en oeuvre de ce type de réacteur est cependant limitée en termes de débit de charge. En effet, un débit de charge trop élevé va conduire au phénomène de blocage du catalyseur contre le collecteur central (ou "pinning" selon la terminologie anglo-saxonne). La force exercée par la charge circulant radialement depuis la périphérie extérieure du lit de catalyseur vers le centre sur les grains de catalyseur, plaque ceux-ci contre la paroi du collecteur central, ce qui augmente la contrainte de frottement qui s'oppose alors au glissement des grains le long de la paroi. Si le courant de charge est suffisamment élevé alors la force de friction qui en résulte est suffisante pour supporter le poids du lit catalytique de sorte que l'écoulement gravitaire des grains de catalyseur cesse, au moins dans certaines régions adjacentes à la paroi du collecteur central. Dans ces régions, les grains de catalyseur sont alors dits "bloqués" ("pinned" selon la terminologie anglo-saxonne) par le débit de gaz et sont maintenus immobiles contre la paroi du collecteur. Le phénomène d'immobilisation des grains de catalyseur est à éviter fortement dans les réacteurs catalytiques, par exemple de reformage catalytique de charge d'hydrocarbures, dans la mesure où il favorise les réactions de désactivation du catalyseur (par exemple par cokage) empêchant à terme la poursuite de l'exploitation du réacteur. Potentiellement lorsque le gâteau de catalyseur devient trop épais le long de la conduite, il est alors nécessaire de réduire le débit de gaz à traiter ou voire arrêter l'unité en vue du décolmatage de ladite conduite.

Par ailleurs, lorsque la perte de charge n'est pas constante sur la hauteur totale du collecteur central, il se crée des chemins préférentiels ("channeling" selon la terminologie anglo-saxonne) dans la section réactionnelle qui alors sont empruntés par le fluide gazeux. Ces chemins préférentiels peuvent se situer, en fonction du sens d'écoulement du fluide gazeux, dans les parties supérieure ou inférieure de la section réactionnelle, et créer des disparités de temps de contact entre le fluide gazeux et le catalyseur dans la section réactionnelle qui peut être à l'origine de baisses de rendement de conversion et/ou de sélectivité de la réaction catalytique.

Il est donc souhaitable pour des questions de distribution homogène de la charge d'équilibrer autant que possible la perte de charge totale entre le lit catalytique et le tube collecteur sur toute la hauteur du lit catalytique. Une méthode employée consiste à ajouter une tube perforé de manière uniforme sur le collecteur central qui produit une perte de charge nette supérieure à celle subie par le fluide gazeux le long de son parcours dans le lit catalytique.

Un but de l'invention est de fournir un dispositif de collecte d'un fluide gazeux pour réacteur radial qui, mis en oeuvre dans ledit réacteur, est moins sujet au phénomène de "pinning" et pour lequel la perte de charge est susceptible d'être contrôlée de manière à limiter les disparités et donc réduire les risques de formation de chemins préférentiels pour le fluide gazeux au sein du lit catalytique.

## Résumé de l'invention

La présente invention a donc pour objet un ensemble de collecte d'un fluide gazeux apte à être disposé dans une section réactionnelle à lit mobile de catalyseur d'un réacteur radial, ledit ensemble de collecte comprenant une grille cylindrique verticale perméable au fluide gazeux et imperméable aux particules de catalyseur et un tube cylindrique vertical supporté par ladite grille et disposé de manière concentrique par rapport à ladite grille. Le tube, perméable au fluide gazeux et imperméable aux particules de catalyseur, comprend une ou plusieurs zones perméables au fluide gazeux comportant une pluralité de trous traversant et une pluralité de zones à perméabilité réduite au fluide gazeux par rapport à la zone perméable au fluide gazeux. Chaque zone à perméabilité réduite présente une porosité plus faible que celle d'une zone dite perméable. La porosité d'une zone est définie par le rapport entre la surface perméable totale de ladite zone et la surface totale développée par ladite zone. Selon l'invention, la porosité d'une zone à perméabilité réduite est comprise entre 0 et 0,005 et étant entendu qu'une zone à perméabilité réduite exclut tout espace compris entre les trous traversant de la ou des zones perméables.

La demanderesse a constaté que la mise en oeuvre d'un tube perforé, présentant une ou plusieurs zones à perméabilité réduite, accolé à la grille pour former un ensemble de collecte permet de contrôler la perte de charge au niveau de ladite grille. En jouant sur la porosité du tube cylindrique, il est ainsi possible de générer différentes pertes charges sur la hauteur de la grille de collecte et donc d'adapter les pertes de charge en fonction de cette hauteur.

De façon surprenante, la demanderesse a constaté que la présence de zones à perméabilité réduite au fluide gazeux formé sur le tube, permet en outre de réduire le blocage du catalyseur contre la grille par rapport à une simple grille ne comportant pas de tube accolée. L'ensemble de collecte selon l'invention permet ainsi de répondre à deux problèmes sans nécessiter de modifier la grille qui est une pièce complexe et fragile de par sa conception.

Dans le contexte de l'invention, l'ensemble de collecte est adapté pour être mis en oeuvre dans des réacteurs à lit catalytique mobile et à circulation radiale de la charge gazeuse, c'est-à-dire dans lesquels le catalyseur est introduit en continu ou en discontinu dans la zone réactionnelle et soutiré en continu ou en discontinu du réacteur.

Selon l'invention, la surface totale développée par les zones à "perméabilité réduite" est généralement comprise entre 1% et 30% de la surface totale développée par le tube.

Selon une mode de réalisation particulier, la section de la grille et du tube est de forme polygonale à au moins 3 côtés, étant entendu que les nombres de côtés des sections de la grille et du tube sont égaux.

Selon une mode de réalisation particulier, la porosité de la zone à perméabilité réduite est égale à 0 de manière à former une zone pleine, étant entendu qu'une zone pleine exclut tout espace plein compris entre les trous traversant d'une zone perméable.

Selon un autre forme de réalisation, la porosité de la zone à perméabilité réduite est différente de 0 et ladite zone comporte des trous traversant.

De préférence, les trous traversant d'une zone à perméabilité réduite sont espacés entre eux d'un pas supérieur au pas entre les trous traversant d'une zone perméable au fluide gazeux.

Selon un autre forme de réalisation, la surface des trous traversant de la zone à perméabilité réduite est inférieure à la surface des trous traversant d'une zone perméable au fluide gazeux.

Les zones à perméabilité réduite peuvent s'étendre en formant un angle $\alpha$ compris entre 0° et 90° par rapport à l'horizontal.

Dans un mode de réalisation préféré, la grille de l'ensemble de collecte est formée par une pluralité de fils verticaux espacés les uns des autres et solidarisés à une pluralité d'anneaux de support horizontaux et le tube cylindrique est solidaire des anneaux de support horizontaux.

L'ensemble de collecte selon l'invention peut prendre une configuration dans laquelle le tube cylindrique est agencé dans l'espace interne délimité par la grille et de manière concentrique par rapport à ladite grille. Alternativement, l'ensemble de collecte selon l'invention est configuré de sorte que la grille soit agencée dans l'espace interne délimité par le tube cylindrique et de manière concentrique par rapport au tube cylindrique.

Un autre aspect de l'invention concerne un réacteur à circulation radiale de fluide gazeux comprenant :

- une enveloppe externe définissant une enceinte s'étendant selon un axe principal vertical et contenant une zone réactionnelle comprenant un lit de particules de catalyseur;
- au moins un moyen d'entrée d'une charge;
- au moins un moyen de sortie de l'effluent produit par la réaction catalytique;
- au moins un moyen d'entrée du catalyseur pour introduire le catalyseur dans la zone réactionnelle;
- au moins un moyen de sortie du catalyseur débouchant dans la zone réactionnelle; et
- un ensemble de collecte selon l'invention, en communication avec le moyen de sortie de l'effluent et dans lequel la grille de l'ensemble de collecte est en contact avec les particules de catalyseur du lit catalytique.

Selon un mode de réalisation, le réacteur comprend en outre une grille cylindrique de distribution du fluide gazeux imperméable au catalyseur disposée entre l'enveloppe externe et l'ensemble de collecte de manière à définir une zone annulaire de distribution comprise entre l'enveloppe et la grille cylindrique de distribution du fluide gazeux, une zone catalytique annulaire comprise entre la grille cylindrique de distribution du fluide gazeux et l'ensemble de collecte et un espace de collecte délimité par l'ensemble de collecte.

Alternativement, un réacteur selon l'invention ne comporte pas de grille de distribution du fluide gazeux mais comprend une pluralité de tubes de distribution du fluide gazeux connectés à une boite de distribution et plongeant dans la zone catalytique annulaire qui est délimitée par l'enceinte et l'ensemble de collecte.

Selon un mode de réalisation alternatif, le réacteur comprend en outre une grille cylindrique de distribution du fluide gazeux imperméable au catalyseur et l'ensemble de collecte est disposé entre l'enveloppe et la grille cylindrique distribution du fluide gazeux de manière à définir une zone annulaire externe de collecte comprise entre l'enveloppe et l'ensemble de collecte, une zone catalytique annulaire comprise entre la grille cylindrique distribution du fluide gazeux et l'ensemble de collecte et un espace de distribution délimité par la grille cylindrique de distribution du fluide gazeux.

Enfin l'invention a également pour objet un procédé de conversion catalytique d'une charge d'hydrocarbures mettant en oeuvre un réacteur selon l'invention, dans lequel :

- on envoie en continu la charge d'hydrocarbures sous forme gazeuse dans un lit catalytique contenu dans le réacteur;
- on met en contact la charge d'hydrocarbures traversant radialement le lit catalytique avec le catalyseur de manière à produire un effluent gazeux; et
- on soutire ledit effluent après son passage à travers l'ensemble de collecte.

Le lit catalytique mis en oeuvre est mobile de sorte que le catalyseur est envoyé en continu ou en discontinu dans la zone réactionnelle et est soutiré en continu ou en discontinu de la zone réactionnelle.

**Description détaillée de l'invention**

Les autres caractéristiques et avantages de l'invention vont apparaître à la lecture de la description qui va suivre, donnée à titre uniquement illustratif et non limitatif, et à laquelle sont annexées :

- la figure 1 qui montre une vue en perspective avec une coupe partielle d'un réacteur à flux radial selon l'art antérieur;
- la figure 2 est vue détaillée en coupe selon un plan perpendiculaire à l'axe vertical d'un ensemble de collecte de la figure 1 ;
- la figure 3 est une vue en plan d'un tube d'un ensemble de collecte selon l'art antérieur;
- les figures 4 à 8 sont des vues en plan de différentes configurations d'un tube selon l'invention;
- la figure 9 est une vue en coupe d'un réacteur selon l'invention, selon un plan perpendiculaire par rapport à l'axe vertical du réacteur;

- la figure 10 est une vue en coupe d'un réacteur selon une autre forme de réalisation, selon un plan perpendiculaire par rapport à l'axe vertical du réacteur.

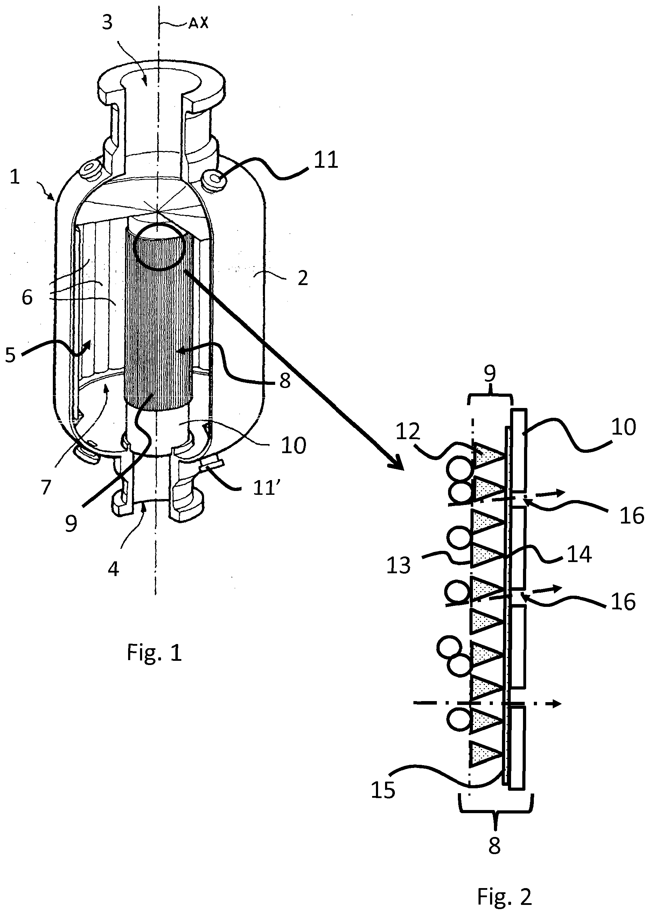

En référence à la figure 1, un réacteur à flux radial 1 selon l'art antérieur se présente extérieurement sous la forme d'une bonbonne formant une enceinte cylindrique 2 s'étendant selon un axe de symétrie vertical AX. L'enceinte 2 comprend dans sa partie supérieure un premier orifice 3 et dans sa partie inférieure un second orifice 4. Les orifices 3 et 4 sont destinés respectivement à l'entrée et à la sortie d'un fluide traversant le réacteur 1. Il est à noter que les fonctions respectives des orifices 3 et 4 peuvent être inversées, c'est-à-dire que l'orifice 4 sert d'orifice d'entrée du fluide et l'orifice 3 est un orifice de sortie de l'effluent réactionnel.

A l'intérieur de ce réservoir cylindrique est agencé un lit catalytique 7 ayant la forme d'un anneau cylindrique vertical limité du côté intérieur par un ensemble de collecte 8 central retenant le catalyseur et du côté extérieur par une grille dite "externe" 5 soit du même type que la grille de l'ensemble de collecte 8, soit par un dispositif consistant en un assemblage d'éléments de grille en forme de coquilles 6 s'étendant longitudinalement, comme représenté sur la figure 1. Ces éléments de grille en forme de coquilles 6 formant des conduits sont également connus sous l'appellation anglo-saxonne de "scallops". Ces conduits 6 sont maintenus par le réservoir et plaqués à la face interne de l'enceinte, parallèlement à l'axe AX, pour former une enveloppe interne sensiblement cylindrique. Les éléments de grille en forme de coquilles 6 sont en communication directe avec le premier orifice 3, par exemple via leur extrémité supérieure, pour recevoir un flux gazeux de charge. Le flux gazeux diffuse à travers la paroi ajourée des conduits 6, pour traverser le lit de particules solides de catalyseur 7 en convergeant radialement vers le centre du réacteur 1. La charge est ainsi mise en contact avec le catalyseur afin de subir des transformations chimiques, par exemple une réaction de reformage catalytique, et produire un effluent de la réaction. L'effluent de la réaction est ensuite collecté par l'ensemble de collecte 8 central qui est ici en communication avec le second orifice 4 du réacteur. L'ensemble de collecte comprend une grille cylindrique 9 et un tube cylindre 10 disposé dans l'espace circonscrit par la grille cylindrique 9. La grille 9 qui agit comme un tamis est conçue de manière à être perméable à un fluide gazeux et imperméable aux particules de catalyseur. Le tube cylindrique 10 est également ajouré et comprend ainsi des trous traversant.

En fonctionnement, le fluide gazeux introduit dans le premier orifice 3 se répartit sur la hauteur du réacteur pour ensuite traverser radialement la grille "externe" 5, puis traverser radialement le lit de particules de catalyseur 7 où il est mis en contact avec le catalyseur afin de produire un effluent qui est par la suite collecté par l'ensemble 8 et évacué par le second orifice 4.

Un tel réacteur peut également fonctionner avec un écoulement gravitaire continu de catalyseur dans le lit catalytique annulaire 7. Dans le cas de la figure 1, le réacteur 1 comprend en outre des moyens d'introduction du catalyseur 11 dans le lit annulaire, disposés dans une partie supérieure du réacteur et des moyens de soutirage du catalyseur 11' qui sont agencés dans une partie inférieure du réacteur.

Le tube 10 est généralement solidarisé à la grille 9 par exemple par boulonnage ou par soudure ou toute autre technique connu de l'homme du métier.

La figure 2 est une vue détaillée en coupe selon un plan perpendiculaire à l'axe vertical AX d'un ensemble de collecte 8 selon l'invention. La grille 9 est constituée d'un assemblage de fils métalliques profilés 12 disposés parallèlement les uns aux autres le long de l'axe vertical de l'ensemble de collecte. Par exemple le profil des fils peut être en V. Comme indiqué sur les figures 2 les fils verticaux 12 présentent une première face 13 et une seconde face opposée 14. Dans le cadre de l'invention, la première face 13 se rapporte à la face qui est en contact avec les particules de catalyseur du lit catalytique lorsque le conduit est mis en oeuvre dans un réacteur radial. La seconde face 14, qui peut être désignée par le vocable "dos", correspond à la face qui n'est pas en contact avec le lit de catalyseur lorsque le conduit est mis en oeuvre dans un réacteur radial. Les fils profilés 12 sont maintenus solidaires entre eux par un ensemble d'anneaux de support métalliques 15 horizontaux soudés à la seconde face 14 des fils verticaux en tout point de contact avec ces derniers. Les anneaux de support 15 sont de préférence régulièrement disposés le long de la hauteur de la grille 9. Grâce à cet agencement des fils verticaux 12 et des anneaux support 15, la grille 9 présente une paroi ajourée sur sa périphérie. La disposition des fils 12 et des anneaux 14 est telle que les ouvertures formées sont aptes à laisser diffuser un fluide gazeux tout en retenant les particules de catalyseur.

L'ensemble de collecte 8 comporte en outre un tube cylindrique 10, disposé par exemple dans l'espace interne délimité par la grille 9, qui est accolé contre ladite grille 9. Plus précisément, le tube 10 est solidarisé du côté "dos" des fils profilés 12 via les anneaux de support 15. Le tube 10 est par ailleurs pourvu d'une pluralité de trous traversant 16 de sorte que le fluide gazeux qui a diffusé radialement à travers la grille puisse également traverser le tube 10.

Il est à noter que selon l'invention, la section de la grille et du tube peut être de forme polygonale à au moins 3 côtés, étant entendu que les nombres de côtés des sections de la grille et du tube sont alors égaux.

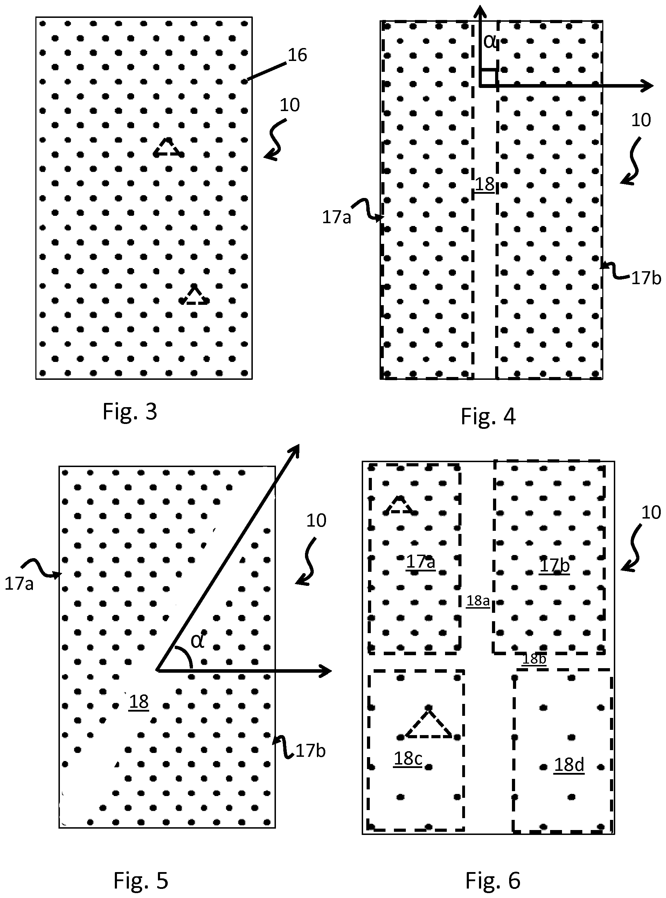

En référence à la figure 3 qui est une vue en plan d'un tube 10 selon l'art antérieur déroulé. Le tube 10 peut être réalisé à partir d'une plaque métallique perforée. Typiquement, un tube selon l'art antérieur comporte une unique zone perméable au fluide gazeux comprenant des trous traversant 16 qui sont disposés uniformément sur sa surface et sur la hauteur dudit tube. Dans l'exemple de la figure 3, les trous 16 sont agencés en formant un réseau en triangle et avec un pas régulier entre les trous adjacents.

La figure 4 montre un premier mode de réalisation d'un tube 10 faisant parti de l'ensemble de collecte selon l'invention. Le tube 10 est composé d'une pluralité de zones 17a et 17b (désignées par les pointillés) pourvues de trous traversant 16 qui sont ainsi perméables au fluide gazeux et une pluralité de zone 18 dites à "perméabilité réduite" au fluide gazeux. Comme montré sur la figure 4, les zones perméables au fluide gazeux 17a et 17b ont la même configuration que celle du tube de la figure 3, à savoir un réseau en triangle de trous traversant 16 et avec un pas régulier entre les trous adjacents. Il est bien entendu possible d'agencer les trous traversant 16 selon un réseau régulier en carré ou en rectangle Alternativement, les trous traversant peuvent être disposés de manière aléatoire. Dans le contexte de l'invention, une zone à perméabilité réduite correspond à une zone dont la porosité, définie par le rapport entre la surface perméable totale de ladite zone (i.e. la surface totale des trous) et la surface totale développée par ladite zone, est comprise entre 0 et 0,005. Une zone dite à "perméabilité réduite" présente nécessairement une porosité plus faible que celle d'une zone dite "perméable". Une zone dite "perméable" présente donc une porosité, qui est le rapport entre la surface perméable totale de ladite zone (i.e. la surface totale des trous) et la surface totale développée par ladite zone, supérieure à 0,0055 et de préférence comprise entre 0,0055 et 0,08, et de manière plus préférée comprise entre 0,0065 et 0,065.

A titre d'exemple, si une zone à perméabilité réduite présente une surface totale développée de 1 $m^2$ et comporte 50 trous traversant de 1,12 cm de rayon, la porosité de ladite zone est égale à :

$$P = (50 \times (\pi \times (1,12.10^{-2})^2)) / (1) = 0,02$$

Dans le mode de réalisation de la figure 4, la zone a perméabilité réduite présente une porosité égale à 0 et correspond à une zone pleine imperméable et exempt de trous traversant, étant entendu qu'une zone pleine imperméable exclut tout espace plein compris entre les trous traversant d'une zone perméable.

Selon l'invention, une zone à "perméabilité réduite" peut s'étendre selon un angle $\alpha$ par rapport à l'horizontal compris entre 0° et 90°. Dans l'exemple de la figure 4, la zone à "perméabilité réduite" 18 est une zone pleine formant une bande pleine continue s'étendant selon l'axe vertical du tube, c'est-à-dire en formant un angle $\alpha$ par rapport à l'horizontal égal à 90°.

Selon l'invention, un tube peut comporter une pluralité de zones perméables au fluide gazeux qui sont séparés par une zone à "perméabilité réduite" au fluide gazeux.

La figure 5 représente une autre forme de configuration du tube 10 de l'ensemble de collecte selon l'invention qui se distingue de celle de la figure 4 en que la zone à "perméabilité réduite" 18 (ici une zone pleine continue) s'étend dans une direction qui forme un angle $\alpha$ par rapport à l'horizontal sensiblement égal à 45°. Lorsque le tube est enroulé, la zone à "perméabilité réduite" 18 forme une hélice ou une portion d'hélice autour du tube.

Dans le cadre de l'invention, le tube de l'ensemble de collecte peut comprendre une unique zone à "perméabilité réduite" formant une hélice continue autour du tube ou, alternativement, une pluralité d'hélices ou une pluralité de portions d'hélice parallèles les unes aux autres autour du tube.

La figure 6 montre une autre forme de réalisation dans laquelle le tube comporte une pluralité de zones perméables au fluide gazeux, ici deux zones perméables 17a, 17b et quatre zones à "perméabilité réduite" 18a, 18b, 18c et 18d. Les zones pleines 18a et 18b se croisent et s'étendent respectivement dans une direction formant un angle $\alpha$ par rapport à l'horizontal de 90° et 0° respectivement. Le tube comporte en outre deux autres zones à "perméabilité réduite" 18c et 18d dans la partie inférieure du tube, qui sont séparées l'une de l'autre par la zone pleine 18a et respectivement des zones perméables 17a et 17b par la zone pleine 18b. La porosité des zones 18c et 18d est réduite par rapport à celle des zones 17a et 17b (dites perméables) en augmentant le pas entre les trous traversant (donc en réduisant la densité de trous traversant) dans les zones.

Ce mode de réalisation illustre le fait qu'il est possible de faire varier la perte de charge localement en fonction de la hauteur du tube de l'ensemble de collecte afin de maintenir un flux de gaz constant le long de l'ensemble de collecte et donc dans le lit catalytique et ainsi limiter la formation de chemins préférentiels ("channeling") dans le lit catalytique.

La réduction de la perméabilité d'une zone entraine une augmentation de la perte de charge au niveau de cette zone. Dans le cas de figure où le fluide gazeux circule selon un mouvement ascendant et radial, celui-ci subit une perte de charge plus importante en tête du réacteur. Pour garantir un flux de gaz uniforme sur la hauteur de la grille de collecte, il est ainsi avantageux de créer une perte de charge dans la section inférieure de la grille grâce au tube cylindrique qui comporte alors des zones à perméabilité réduite dans sa section inférieure tandis que sa section supérieure peut être dépourvue de zone à perméabilité réduite.

L'agencement des zones perméables et des zones à perméabilité réduite est inversée lorsque l'ensemble de collecte est utilisé dans un réacteur dans lequel le fluide gazeux est injecté en tête dudit réacteur et l'effluent réactionnel est récupéré en fond du réacteur. Dans une telle situation, la perte de charge est plus importante en fond du réacteur qu'en tête du réacteur.

Un autre avantage de la mise en oeuvre d'un ensemble de collecte selon l'invention dans un réacteur à lit mobile de catalyseur décrit en référence à la figure 1 est de permettre réduire la taille d'un tel réacteur. En effet il est envisageable de réduire l'espace de distribution du fluide gazeux compris entre le réacteur et la grille de distribution, même au prix d'une augmentation de la perte de charge subie par le fluide dans cet espace qui peut être reprise au niveau de l'ensemble de collecte selon l'invention en adaptant la porosité au niveau du tube cylindrique.

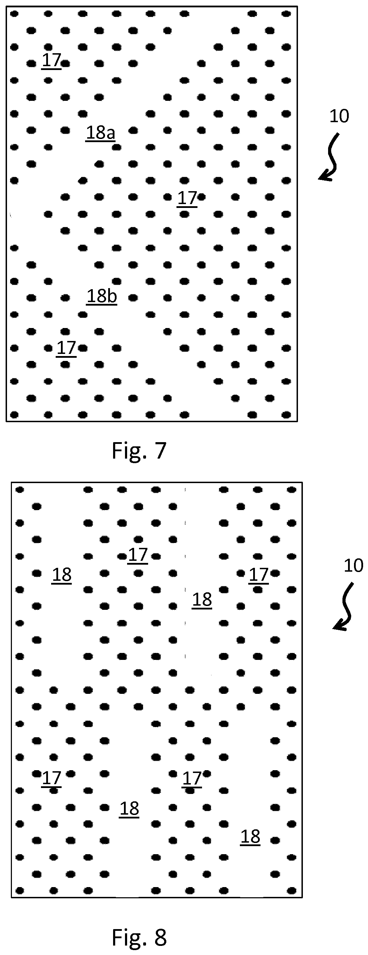

La figure 7 représente un autre mode de configuration d'un tube 10 de l'ensemble de collecte selon l'invention dans lequel les zones 18a et 18b à "perméabilité réduite" par rapport aux zones perméables 17, qui sont ici des zones pleines, s'étendent respectivement en formant un angle $\alpha$ et - $\alpha$ par rapport à l'horizontal et se rencontrent à l'une de leur extrémités de sorte à former une bande pleine continue de forme en "V". Alternativement à ce mode de réalisation, deux zones 18a et 18b à "perméabilité réduite" peuvent se croiser entre eux selon une forme en X avec une première zone à "perméabilité réduite" inclinée d'un angle $\alpha$ par rapport à l'horizontal et une seconde zone à "perméabilité réduite" inclinée d'un angle -$\alpha$ par rapport à l'horizontal. Ces modes de réalisation peuvent être déclinés de sorte que le tube soit garni d'une pluralité de zones à "perméabilité réduite" de forme en "V" et/ ou en "X" autour dudit tube.

Un autre mode de réalisation du tube de l'ensemble de collecte selon l'invention est représenté en plan dans la figure 8. Le tube comprend une pluralité de zones perméables au fluide gazeux et une pluralité de zones à "perméabilité réduite" 18 au fluide gazeux qui sont disposés de telle manière qu'une partie des zones à "perméabilité réduite" sont situées dans la moitié supérieure du tube et une autre partie des zones à "perméabilité réduite" sont situées dans la moitié inférieure du tube. Dans la forme de réalisation de la figure 8, les zones à "perméabilité réduite" 18 sont en outre arrangées alternativement dans la moitié supérieure et dans la moitié inférieure du tube.

Quelle que soit la disposition des zones à "perméabilité réduite", celles-ci s'étendent de préférence sur une distance comprise entre 10% et 100% de la hauteur du tube et de manière plus préférée comprise entre 50% à 100% de la hauteur du tube.

Le nombre de zones à "perméabilité réduite" au fluide gazeux et leur dimension peuvent être choisis de manière à ce que la surface totale développée par lesdites zones à "perméabilité réduite" soit comprise entre 1% et 30% de la surface totale développée par le tube.

Il est à noter par ailleurs que dans le cadre de l'invention il est possible d'utiliser comme leviers pour modifier la porosité des zones à "perméabilité réduite" la taille de la surface des trous traversant et/ou le nombre de trous. Selon l'invention, les trous traversant peuvent présenter une section de forme quelconque, par exemple circulaire, triangle, carré ou encore rectangle.

La demanderesse a constaté de manière surprenante que, pour une même vitesse de gaz traversant l'ensemble de collecte (grille + tube perforé), la mise en oeuvre d'un tube comprenant des zones à "perméabilité réduite" au fluide gazeux permet de limiter l'épaisseur du gâteau de particules qui est bloqué par le flux de gaz par rapport à un ensemble de collecte de l'art antérieur constitué par une grille à laquelle est accolée un tube ne comportant pas de zones à "perméabilité réduite". La présence de ces zones à "perméabilité réduite" permet ainsi de générer des zones sur la grille où les particules sont moins plaquées et sur lesquelles le gâteau de solide ne peut pas se développer avec pour effet de limiter l'épaisseur du gâteau formé sur la grille. Ainsi en réduisant la quantité de catalyseur "bloqué" par le gâteau, on réduit la part de catalyseur "inactif" et donc on améliore les performances catalytiques du réacteur.

L'invention se rapporte également à un procédé de traitement catalytique d'une charge d'hydrocarbures dans un réacteur radial à lit mobile de catalyseur. Le réacteur selon l'invention comprend :

- une enveloppe définissant une enceinte s'étendant selon un axe principal vertical et contenant une zone réactionnelle à lit de particules de catalyseur,
- au moins un moyen d'entrée d'une charge,
- au moins un moyen de sortie de l'effluent produit par la réaction catalytique,
- au moins un moyen d'entrée du catalyseur pour introduire le catalyseur dans la zone réactionnelle;
- au moins un moyen de sortie du catalyseur débouchant dans la zone réactionnelle; et
- un ensemble de collecte de l'effluent selon l'invention disposé dans la zone réactionnelle et en communication avec le moyen de sortie de l'effluent.

Par ailleurs le réacteur peut inclure optionnellement une grille dite "de distribution du fluide gazeux" perméable aux gaz et imperméable aux particules de catalyseur, qui est disposée dans l'enceinte et de manière concentrique par rapport à l'ensemble de collecte et la grille de l'ensemble de collecte étant en contact avec les particules de catalyseur du lit catalytique.

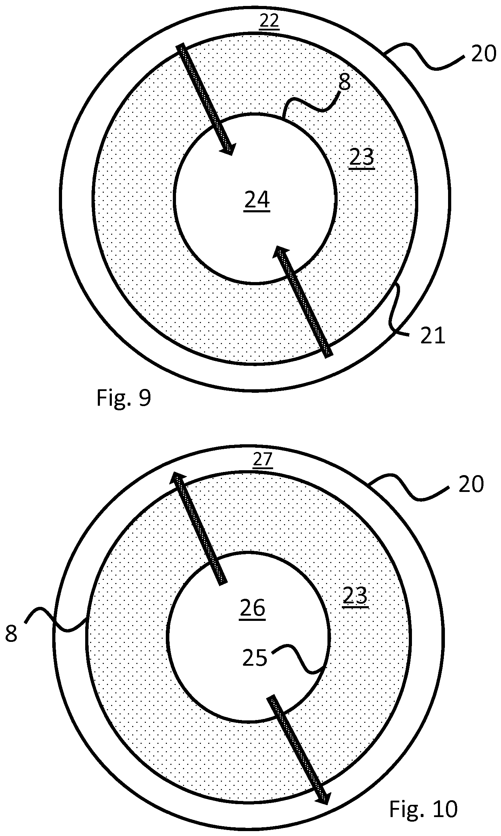

Un mode de réalisation du réacteur selon l'invention, représenté à la figure 9, est un réacteur à circulation radiale centripète (i.e. le flux gazeux circule depuis la périphérie de l'enceinte vers le centre de l'enceinte). Le réacteur comprend une enveloppe 20 qui délimite une enceinte dans laquelle sont agencés une grille cylindrique dite "externe" 21 perméable aux gaz et imperméable au catalyseur et un ensemble de collecte 8 selon l'invention. La grille externe 21 est disposée entre l'enveloppe 20 et l'ensemble de collecte 8 de manière concentrique par rapport l'ensemble de collecte. L'ensemble de collecte 8 est arrangé de sorte que sa grille de collecte (non représenté) soit en contact avec le catalyseur. Dans une telle configuration, le réacteur comprend une zone annulaire "externe" 22 comprise entre l'enveloppe 20 et la grille 21 dite "externe", une zone catalytique annulaire 23 comprise entre la grille 21 dite "externe" et l'ensemble de collecte 8 et un espace de collecte cylindrique 24 délimité par l'ensemble de collecte 8. La grille 21 dite "externe" peut prendre la forme d'une plaque perforée ou d'une grille formée par un maillage de fils et de tiges métalliques profilés ou encore d'un assemblage d'éléments de grille en forme de coquille (ou "scallop" selon la terminologie anglo-saxonne). En fonctionnement, la charge gazeuse est injectée soit par le fond soit par le sommet du réacteur dans la zone annulaire 22 de distribution puis passe à travers la grille 21 dite "externe" et traverse ensuite de manière sensiblement radiale le lit de particules de catalyseur de la zone catalytique annulaire 23. Dans la zone catalytique annulaire 23 le fluide gazeux est mis en contact avec le catalyseur pour produire un effluent réactionnel gazeux qui est collecté dans l'espace 24 de l'ensemble de collecte 8 et qui est ensuite soutiré soit au sommet du réacteur (lorsque la charge est introduite en fond du réacteur) soit en fond du réacteur (lorsque la charge est introduite par le sommet du réacteur).

Selon une mode de réalisation alternatif (non représenté), le réacteur ne comporte pas de grille cylindrique 21 mais une pluralité de tubes de distribution, connectés à une boite de distribution (externe ou interne au réacteur) et plongeant dans la zone réactionnelle, qui permettent de distribuer la charge gazeuse dans la zone catalytique 23.

Un autre mode de réalisation du réacteur selon l'invention est représenté à la figure 10 qui met en oeuvre un ensemble de collecte 8 selon l'invention disposée entre l'enceinte 20 et une grille cylindrique de distribution 25 du fluide gazeux qui est arrangé au centre de l'enceinte 20. La grille de 25 peut être du même type que la grille 21 décrite en référence à la figure 9. Il est à noter que dans cette configuration, l'ensemble de collecte est disposée dans le réacteur de sorte que la grille de collecte (non représentée sur la figure 10) soit directement en contact avec le catalyseur.

Comme indiqué sur la figure 10, le réacteur comporte une zone cylindrique 26 dans laquelle circule le fluide gazeux, une zone catalytique annulaire 23 délimitée par la grille de distribution 25 et l'ensemble de collecte 8 et une zone cylindrique de collecte externe 27 définie entre l'enceinte 20 et l'ensemble de collecte 8. Cette configuration de réacteur est adaptée pour traiter un fluide gazeux qui circule de manière radiale selon une direction centrifuge, comme représenté par les flèches sur la figure 10. La charge gazeuse est injectée soit par le fond soit par le sommet du réacteur dans la zone cylindrique 26 de distribution puis passe à travers la grille 25 et traverse ensuite de manière sensiblement radiale le lit de particules de catalyseur de la zone catalytique annulaire 23. Dans la zone catalytique annulaire 23 le fluide gazeux est mis en contact avec le catalyseur pour produire un effluent réactionnel gazeux qui est collecté dans l'espace de collecte 27 compris entre l'enceinte et de l'ensemble de collecte 8. L'effluent réactionnel est ensuite soutiré soit au sommet du réacteur (lorsque la charge est introduite en fond du réacteur) soit en fond du réacteur (lorsque la charge est introduite par le sommet du réacteur).

Selon un mode de réalisation non représenté, un réacteur selon l'invention ne comporte pas de grille de distribution du fluide gazeux mais comprend une pluralité de tubes de distribution du fluide gazeux connectés à une boite de distribution et plongeant dans la zone catalytique annulaire qui est délimitée par l'enceinte et l'ensemble de collecte.

Le réacteur selon l'invention peut être un réacteur à lit catalytique mobile où le catalyseur est introduit en continu ou en discontinu dans le réacteur et soutiré dudit réacteur en continu ou en discontinu respectivement.

Le réacteur et le procédé selon l'invention peuvent être appliqués pour réaliser des réactions à circulation radiale de fluide gazeux comme par exemple une réaction de reformage catalytique d'une coupe d'hydrocarbures, une isomérisation squelettale des oléfines, la métathèse pour la production de propylène, une réaction d'oligocraquage.

## Revendications

1. Ensemble de collecte (8) d'un fluide gazeux apte à être disposé dans une section réactionnelle à lit mobile de catalyseur d'un réacteur radial, ledit ensemble de collecte comprenant une grille cylindrique verticale (9) perméable au fluide gazeux et imperméable aux particules de catalyseur et un tube cylindrique (10) vertical supporté par ladite grille (9) et disposé de manière concentrique par rapport à ladite grille, ledit tube étant perméable au fluide gazeux et imperméable aux particules de catalyseurs, dans lequel le tube (10) comprend une ou plusieurs zones (17a, 17b) perméables au fluide gazeux comportant une pluralité de trous traversant et une pluralité de zones (18a, 18b, 18 c, 18d) à "perméabilité réduite" au fluide gazeux par rapport à la zone perméable, chaque zone à perméabilité réduite ayant une porosité plus faible que celle d'une zone dite perméable, la porosité des zones étant définie comme étant le rapport entre la surface perméable totale de ladite zone et la surface totale développée par ladite zone, et dans lequel la porosité d'une zone à perméabilité réduite est comprise entre 0 et 0,005 et étant entendu qu'une zone à perméabilité réduite exclut tout espace compris entre les trous traversant de la zone perméable.

2. Ensemble de collecte selon la revendication 1, dans lequel la surface totale développée par les zones à "perméabilité réduite" est comprise entre 1% et 30% de la surface totale développée par le tube.

3. Ensemble de collecte selon l'une des revendications 1 à 2, dans lequel la section de la grille (9) et du tube (10) est de forme polygonale à au moins 3 côtés, étant entendu que les nombres de côtés des sections de la grille et du tube sont égaux.

4. Ensemble de collecte selon l'une des revendications précédentes, dans lequel la porosité d'une zone à perméabilité réduite est égale à 0 de manière à former une zone pleine, étant entendu qu'une zone pleine exclut tout espace plein compris entre les trous traversant d'une zone perméable.

5. Ensemble de collecte selon l'une des revendications 1 à 3, dans lequel la porosité d'une zone à perméabilité réduite est différente de 0 et ladite zone comporte des trous traversant.

6. Ensemble de collecte selon la revendication 5, dans lequel les trous traversant d'une zone à perméabilité réduite sont espacés entre eux d'un pas supérieur au pas entre les trous traversant de la zone perméable au fluide gazeux.

7. Ensemble de collecte selon les revendications 5 ou 6, dans lequel la surface des trous traversant d'une zone à "perméabilité réduite" est inférieure à la surface des trous traversant de la zone perméable au fluide gazeux.

8. Ensemble de collecte selon l'une des revendications précédentes, dans lequel les zones à "perméabilité réduite" s'étendent en formant un angle $\alpha$ compris entre 0° et 90° par rapport à l'horizontal.

9. Ensemble de collecte selon l'une des revendications précédentes, dans lequel la grille (9) est formée par une pluralité de fils verticaux (12) espacés les uns des autres et solidarisés à une pluralité d'anneaux de support horizontaux (15) et le tube cylindrique (10) est solidaire des anneaux de support horizontaux (15).

10. Ensemble de collecte selon l'une des revendications précédentes dans lequel le tube cylindrique (10) est agencé dans l'espace interne délimité par la grille et de manière concentrique par rapport à ladite grille (9).

11. Ensemble de collecte selon l'une des revendications 1 à 9, dans lequel la grille (9) est agencée dans l'espace interne délimité par le tube cylindrique (10) et de manière concentrique par rapport au tube cylindrique (10).

12. Réacteur à circulation radiale de fluide gazeux comprenant :

  • une enveloppe externe (20) définissant une enceinte s'étendant selon un axe principal vertical et contenant une zone réactionnelle comprenant un lit de particules de catalyseur;

• au moins un moyen d'entrée d'une charge;

• au moins un moyen de sortie de l'effluent produit par la réaction catalytique;

• au moins un moyen d'entrée du catalyseur pour introduire le catalyseur dans la zone réactionnelle;

• au moins un moyen de sortie du catalyseur débouchant dans la zone réactionnelle; et

• un ensemble de collecte (8) selon l'une des revendications précédentes, en communication avec le moyen de sortie de l'effluent et la grille de l'ensemble de collecte étant en contact avec les particules de catalyseur du lit catalytique.

13. Réacteur selon la revendication 12, comprenant en outre une grille cylindrique de distribution du fluide gazeux (21) imperméable au catalyseur disposée entre l'enveloppe externe et l'ensemble de collecte de manière à définir une zone annulaire de distribution (22) comprise entre l'enveloppe (20) et la grille cylindrique de distribution (21) du fluide gazeux, une zone catalytique annulaire (23) comprise entre la grille cylindrique de distribution (21) du fluide gazeux et l'ensemble de collecte (8), et un espace de collecte (24) délimité par l'ensemble de collecte (8).

14. Réacteur selon la revendication 12, comprenant en outre une grille cylindrique de distribution du fluide gazeux (25) imperméable au catalyseur et dans lequel l'ensemble de collecte (8) est disposé entre l'enveloppe (2) et la grille cylindrique distribution du fluide gazeux (25), de manière à définir une zone annulaire externe de collecte (27) comprise entre l'enveloppe (20) et l'ensemble de collecte (8), une zone catalytique annulaire (23) comprise entre la grille cylindrique distribution du fluide gazeux (5) et l'ensemble de collecte (8) et un espace de distribution (26) délimité par la grille cylindrique de distribution du fluide gazeux (25).

15. Procédé de conversion catalytique d'une charge d'hydrocarbures mettant en oeuvre un réacteur selon l'une des revendications 12 à 14, dans lequel:

• on envoie en continu la charge d'hydrocarbures sous forme gazeuse dans un lit catalytique contenu dans le réacteur;

• on met en contact la charge d'hydrocarbures traversant radialement le lit catalytique avec le catalyseur de manière à produire un effluent gazeux; et

• on soutire ledit effluent après son passage à travers l'ensemble de collecte.

16. Procédé selon la revendication 15, dans lequel le lit catalytique est mobile et le catalyseur est introduit en continu ou en discontinu dans le réacteur et soutiré dudit réacteur en continu ou en discontinu, respectivement.

**Patentansprüche**

1. Anordnung zum Sammeln (8) eines gasförmigen Fluids, die geeignet ist, in einem Reaktionsabschnitt mit bewegtem Katalysatorbett eines Radialreaktors angeordnet zu werden, wobei die Anordnung zum Sammeln ein vertikales zylindrisches Gitter (9), das für das gasförmige Fluid durchlässig und für die Katalysatorteilchen undurchlässig ist, und ein vertikales zylindrisches Rohr (10) aufweist, das von dem Gitter (9) getragen und konzentrisch zu dem Gitter angeordnet ist, wobei das Rohr für das gasförmige Fluid durchlässig und für die Katalysatorteilchen undurchlässig ist, wobei das Rohr (10) einen oder mehrere Bereiche (17a, 17b) aufweist, die für das gasförmige Fluid durchlässig sind, die mehrere Durchgangslöcher und mehrere Bereiche (18a, 1Bb, 16c, 18d) mit "verminderter Durchlässigkeit" für das gasförmige Fluid im Vergleich zu dem durchlässigen Bereich aufweisen, wobei jeder Bereich mit verminderter Durchlässigkeit eine geringere Porosität als jene eines durchlässigen Bereichs aufweist, wobei die Porosität der Bereiche als das Verhältnis zwischen der durchlässigen Gesamtoberfläche des Bereichs und der Gesamtabwicklungsoberfläche, definiert wird, und wobei die Porosität eines Bereichs mit verminderter Permeabilität zwischen 0 und 0,005 liegt, und mit der Maßgabe, dass ein Bereich mit verringerter Durchlässigkeit jeden Raum ausschließt, der zwischen den Durchgangslöchern des durchlässigen Bereichs liegt.

2. Anordnung zum Sammeln nach Anspruch 1, wobei die Gesamtabwicklungsfläche, der Bereiche mit "verminderter Durchlässigkeit" zwischen 1 % und 30 % der Gesamtabwicklungsoberfläche, des Rohres beträgt.

3. Anordnung zum Sammeln nach einem der Ansprüche 1 bis 2, wobei der Bereich des Gitters (9) und des Rohrs (10) eine polygonale Form mit mindestens 3 Seiten aufweist, mit der Maßgabe, dass die Anzahl an Seiten der Bereiche des Gitters und des Rohrs gleich sind.

4. Anordnung zum Sammeln nach einem der vorhergehenden Ansprüche, wobei die Porosität eines Bereichs mit verminderter Durchlässigkeit gleich 0 ist, derart, um einen vollen Bereich zu bilden, mit der Maßgabe, dass ein voller Bereich jeden vollen Raum ausschließt, der zwischen den Durchgangslöchern eines durchlässigen Bereichs liegt.

5. Anordnung zum Sammeln nach einem der Ansprüche 1 bis 3, wobei die Porosität eines Bereichs mit verminderter Durchlässigkeit von 0 verschieden ist und der Bereich Durchgangslöcher aufweist.

6. Anordnung zum Sammeln nach Anspruch 5, wobei die Durchgangslöcher eines Bereichs mit verminderter Durchlässigkeit um einen Abstand voneinander beabstandet sind, der größer als der Abstand zwischen den Durchgangslöchern des Bereichs ist, der für das gasförmige Fluid durchlässig ist.

7. Anordnung zum Sammeln nach Anspruch 5 oder 6, wobei die Oberfläche der Durchgangslöcher eines Bereichs mit "verminderter Durchlässigkeit" kleiner als die Oberfläche der Durchgangslöcher des Bereichs ist, der für das gasförmige Fluid durchlässig ist.

8. Anordnung zum Sammeln nach einem der vorhergehenden Ansprüche, wobei sich die Bereiche mit "verminderter Durchlässigkeit" erstrecken, indem sie einen Winkel $\alpha$ bilden, der zwischen 0° und 90° zur Horizontalen liegt.

9. Anordnung zum Sammeln nach einem der vorhergehenden Ansprüche, wobei das Gitter (9) aus mehreren vertikalen Drähten (12) gebildet ist, die voneinander beabstandet sind und mit mehreren horizontalen Stützringen (15) fest verbunden sind, und das zylindrische Rohr (10) mit den horizontalen Stützringen (15) fest verbunden ist.

10. Anordnung zum Sammeln nach einem der vorhergehenden Ansprüche, wobei das zylindrische Rohr (10) in dem Innenraum, der von dem Gitter begrenzt ist, und konzentrisch zu dem Gitter (9) angeordnet ist.

11. Anordnung zum Sammeln nach einem der Ansprüche 1 bis 9, wobei das Gitter (9) in dem Innenraum, der von dem zylindrischen Rohr (10) begrenzt ist, und konzentrisch zu dem zylindrischen Rohr (10) angeordnet ist.

12. Reaktor mit radialer Zirkulation eines gasförmigen Fluids, umfassend:

    • eine äußere Hülle (20), die einen Behälter definiert, der sich entlang einer vertikalen Hauptachse erstreckt und eine Reaktionszone enthält, die ein Katalysatorteilchenbett aufweist;
    • mindestens ein Einlassmittel einer Beschickung;
    • mindestens ein Auslassmittel des Abstroms, der durch die katalytische Reaktion erzeugt wird;
    • mindestens ein Einlassmittel des Katalysators, um den Katalysator in die Reaktionszone einzuführen;

    • mindestens ein Auslassmittel des Katalysators, der in die Reaktionszone mündet; und
    • eine Anordnung zum Sammeln (8) nach einem der vorhergehenden Ansprüche in Kommunikation mit dem Auslassmittel des Abstroms und wobei das Gitter der Anordnung zum Sammeln in Kontakt mit den Katalysatorteilchen desKatalysatorbetts ist.

13. Reaktor nach Anspruch 12, ferner umfassend ein zylindrisches Gitter zum Verteilen des gasförmigen Fluids (21), das für den Katalysator undurchlässig ist, das zwischen der äußeren Hülle und der Anordnung zum Sammeln derart angeordnet ist, um einen ringförmigen Verteilungsbereich (22) zu bilden, der zwischen der Hülle (20) und dem zylindrischen Gitter zum Verteilen (21) des gasförmigen Fluids liegt, eine ringförmige katalytische Zone (23), die zwischen dem zylindrischen Gitter zum Verteilen (21) des gasförmigen Fluids und der Anordnung zum Sammeln (8) liegt, und einen Sammelraum (24), der durch die Anordnung zum Sammeln (8) begrenzt ist.

14. Reaktor nach Anspruch 12, ferner umfassend ein zylindrisches Gitter zum Verteilen des gasförmigen Fluids (25), das für den Katalysator undurchlässig ist, und wobei die Anordnung zum Sammeln (8) zwischen der Hülle (2) und dem zylindrischen Gitter zum Verteilen des gasförmigen Fluids (25) derart angeordnet ist,um einen ringförmigen äußeren Sammelbereich (27) zu bilden, der zwischen der Hülle (20) und der Anordnung zum Sammeln (8) liegt, eine ringförmige katalytische Zone (23), die zwischen dem zylindrischen Gitter zum Verteilen des gasförmigen Fluids (5) und der Anordnung zum Sammeln (8) liegt, und einen Verteilungsraum (26), der durch das zylindrische Gitter zum Verteilen des gasförmigen Fluids (25) begrenzt ist.

15. Verfahren zur katalytischen Umwandlung einer Kohlenwasserstoffbeschickung, das einen Reaktor nach einem der Ansprüche 12 bis 14 einsetzt, wobei:

    • die Kohlenwasserstoffbeschickung kontinuierlich in Form eines Gases in ein Katalysatorbett geschickt wird, das in dem Reaktor enthalten ist,
    • die Kohlenwasserstoffbeschickung, die das Katalysatorbett radial durchquert, mit dem Katalysator derart in Kontakt gebracht wird, um einen gasförmigen Abstrom zu erzeugen und
    • der Abstrom nach seinem Durchgang durch die Anordnung zum Sammeln abgezogen wird.

16. Verfahren nach Anspruch 15, wobei das Katalysatorbett beweglich ist und der Katalysator kontinuierlich oder diskontinuierlich in den Reaktor eingeführt wird und aus dem Reaktor jeweils kontinuierlich oder diskontinuierlich abgezogen wird.

## Claims

1. A collector assembly (8) for a gaseous fluid suitable for being disposed in a reaction section with a moving bed of catalyst of a radial reactor, said collector assembly comprising a vertical cylindrical screen (9) which is permeable to gaseous fluid and impermeable to particles of catalyst, and a vertical cylindrical tube (10) which is supported by said screen (9) and disposed in a concentric manner with respect to said screen, said tube being permeable to gaseous fluid and impermeable to particles of catalyst, in which the tube (10) comprises one or more zones (17a, 17b) which are permeable to gaseous fluid comprising a plurality of through holes and a plurality of zones (18a, 18b, 18c, 18d) with a "reduced permeability" to gaseous fluid compared with the permeable zone, each zone with reduced permeability having a lower porosity than that of a zone which is said to be permeable, the porosity of the zones being defined as the ratio between the total permeable surface area of said zone and the total developed surface area of said zone, and in which the porosity of a zone with reduced permeability is in the range 0 to 0.005, it being understood that a zone with reduced permeability excludes any space taken up by the through holes of the permeable zone.

2. The collector assembly according to claim 1, in which the total developed surface area of the zones with "reduced permeability" is in the range 1 % to 30% of the total developed surface area of the tube.

3. The collector assembly according to claim 1 or claim 2, in which the section of the screen (9) and the tube (10) is polygonal in shape with at least 3 sides, it being understood that the number of sides of the sections of the screen and tube are equal.

4. The collector assembly according to one of the preceding claims, in which the porosity of a zone with reduced permeability is equal to 0 in a manner such as to form a solid zone, it being understood that a solid zone excludes any solid space taken up by the through holes of a permeable zone.

5. The collector assembly according to one of claims 1 to 3, in which the porosity of a zone with reduced permeability is other than 0 and said zone comprises through holes.

6. The collector assembly according to claim 5, in which the through holes of a zone with reduced permeability are spaced from each other by a pitch which is higher than the pitch between the through holes of a zone which is permeable to gaseous fluid.

7. The collector assembly according to claim 5 or claim 6, in which the surface area of the through holes of a zone with "reduced permeability" is smaller than the surface area of the through holes of a zone which is permeable to gaseous fluid.

8. The collector assembly according to one of the preceding claims, in which the zones with "reduced permeability" are extended, forming an angle α in the range 0° to 90° with respect to the horizontal.

9. The collector assembly according to one of the preceding claims, in which the screen (9) is formed by a plurality of vertical wires (12) spaced from each other and attached to a plurality of horizontal support rings (15), and the cylindrical tube (10) is attached to the horizontal support rings (15).

10. The collector assembly according to one of the preceding claims, in which the cylindrical tube (10) is disposed in the internal space delimited by the screen and is concentric with respect to said screen (9).

11. The collector assembly according to one of claims 1 to 9, in which the screen (9) is disposed in the internal space delimited by the cylindrical tube (10) and is concentric with respect to the cylindrical tube (10).

12. A reactor with a radial movement of gaseous fluid, comprising:

    • an outer envelope (20) defining a vessel extending along a principal vertical axis and containing a reaction zone comprising a bed of particles of catalyst;
    • at least one inlet means for a feed;
    • at least one outlet means for effluent produced by the catalytic reaction;
    • at least one inlet means for catalyst in order to introduce the catalyst into the reaction zone;
    • at least one outlet means for catalyst, opening into the reaction zone; and
    • a collector assembly (8) in accordance with one of the preceding claims, in communication with the effluent outlet means and the screen of the collector assembly is in contact with the particles of catalyst of the catalytic bed.

13. The reactor according to claim 12, further comprising a cylindrical screen (21) for distribution of gaseous fluid which is impermeable to catalyst, disposed between the external envelope and the collector assembly in a manner such as to define an annular distribution zone (22) comprised between the envelope (20) and the cylindrical gaseous fluid distribution screen (21), an annular catalytic zone (23) comprised between the cylindrical gaseous fluid distribution screen (21) and the collector assembly (8), and a collector space (24) delimited by the collector assembly (8).

14. The reactor according to claim 12, further comprising a cylindrical gaseous fluid distribution screen (25) which is impermeable to catalyst and in which the collector assembly (8) is disposed between the envelope (20) and the cylindrical gaseous fluid distribution screen (25) in a manner such as to define an outer annular collector zone (27) comprised between the envelope (20) and the collector assembly (8), an annular catalytic zone (23) comprised between the cylindrical gaseous fluid distribution screen (25) and the collector assembly (8) and a distribution space (26) delimited by the cylindrical gaseous fluid distribution screen (25).

15. A process for the catalytic conversion of a hydrocarbon feed using a reactor in accordance with one of claims 12 to 14, in which:

• the hydrocarbon feed in the gaseous form is continuously supplied to a catalytic bed contained in the reactor;
• the hydrocarbon feed passing radially through the catalytic bed is brought into contact with the catalyst in a manner such as to produce a gaseous effluent; and
• said effluent is withdrawn after it has passed through the collector assembly.

16. The process according to claim 15, in which the catalytic bed is a moving bed and the catalyst is introduced into the reactor continuously or discontinuously and respectively continuously or discontinuously withdrawn from said reactor.

Fig. 1

Fig. 2

Fig. 3

Fig. 4

Fig. 5

Fig. 6

Fig. 7

Fig. 8

Fig. 9

Fig. 10